Europäisches Patentamt

European Patent Office

Office européen des brevets

(19)

(11) Numéro de publication: **0 236 229**
**A1**

(12) **DEMANDE DE BREVET EUROPEEN**

(21) Numéro de dépôt: **87400457.5**

(22) Date de dépôt: **02.03.87**

(51) Int. Cl.⁴: **A 61 N 1/05**

(30) Priorité: **04.03.86 FR 8603037**

(43) Date de publication de la demande:
**09.09.87  Bulletin  87/37**

(84) Etats contractants désignés: **DE GB IT NL**

(71) Demandeur: **Amiel, Alain Pierre**
**1 Rue de Poitiers**
**F-86130 Jaunay Clan  (FR)**

**Besson, Jacques**
**29 Route de Gencay**
**F-86000 Poitiers  (FR)**

(72) Inventeur: **Amiel, Alain Pierre**
**1 Rue de Poitiers**
**F-86130 Jaunay Clan  (FR)**

**Besson, Jacques**
**29 Route de Gencay**
**F-86000 Poitiers  (FR)**

(74) Mandataire: **Rataboul, Michel**
**Cabinet Michel Rataboul 69, rue de Richelieu**
**F-75002 Paris  (FR)**

(54) **Electrode myocardique à élément conducteur distinct.**

(57) Les électrodes myocardiques jouent deux rôles : envoyer des impulsions électriques au muscle cardiaque et recueillir le signal cardiaque naturel pour ajuster les impulsions. Pour que l'envoi des impulsions soit le meilleur possible, il faut que l'électrode soit de petite étendue alors que pour avoir un bon recueil, il faut au contraire que l'électrode soit vaste.

Pour concilier ces deux impératifs contradictoires, l'électrode a une coupelle (1) sur laquelle sont prévus des éléments conducteurs (6) distincts et situés hors de la surface de la coupelle, soit en étant en relief, comme représenté, soit en étant en creux. Ces éléments (6) sont en forme de spirale exponentielle afin de bénéficier de la caractéristique de cette spirale selon laquelle son entropie est nulle. Cet ensemble permet de concentrer les lignes de force du champ électrique issu des éléments (6) selon un volume situé au-delà du bord (7), c'est-à-dire là où doit se situer la partie du coeur devant recevoir les impulsions. La forme évasée de la coupelle 1 donne à l'électrode l'étendue necessaire à un bon recueil.

**Description**

ELECTRODE MYOCARDIQUE A ELEMENT CONDUCTEUR DISTINCT

On connaît depuis longtemps les électrodes myocardiques, destinées à être maintenues contre la paroi du muscle cardiaque pour stimuler son fonctionnement au moyen d'impulsions électriques.

Afin que ces impulsions soient correctement adaptées au fonctionnement du coeur, il est impératif de recueillir le "signal" cardiaque pour que l'appareil générateur d'impulsions, le stimulateur, ajuste ses paramètres de fonctionnement ou d'arrêt, de rythme, d'intensité etc.

Ces deux fonctions de stimulation et de recueil sont donc complémentaires et l'une ne doit pas être sacrifiée à l'autre. Or, elles imposent des conditions de fonctionnement difficilement compatibles : le seuil de stimulation, c'est-à-dire l'énergie minimale requise pour stimuler le muscle cardiaque, doit être aussi bas que possible pour nécessiter peu d'énergie. Ce seuil dépend de la surface de contact entre l'électrode et la paroi cardiaque, donc plus cette surface est petite, plus le seuil de stimulation est bas et plus la consommation d'énergie est faible. En revanche, le recueil est d'autant meilleur que la surface de contact entre l'électrode et la paroi cardiaque est grande.

Jusqu'à ce jour, on ne détermine les dimensions de l'électrode qu'en se résignant à un compromis qui, par définition, ne répond parfaitement à aucune des deux exigences.

On a déjà tenté d'améliorer ces conditons. Ainsi, on connaît le document FR-A-2 565 111 qui décrit une électrode ayant une tête concave dont la surface possède des facettes orientées de telle sorte que les lignes de force du champ électrique sont focalisées en un segment axial.

La présente invention apporte une solution nouvelle au problème de la stimulation cardiaque et permet de concilier les impératifs contradictoires sans sacrifier ni le seuil de stimulation, ni la qualité du recueil.

A cette fin, l'invention a pour objet une électrode myocardique, du type comprenant une pièce qui est conductrice au moins en partie et qui doit être placée en regard d'une paroi cardiaque, caractérisée en ce qu'elle présente au moins un élément conducteur distinct de la pièce proprement dite, qui s'étend hors de la surface de la pièce devant être en regard de la paroi cardiaque et qui est disposé selon au moins une spirale.

Selon d'autres caractéristiques de l'invention :
- la, respectivement chaque, spirale est de type exponentiel, c'est-à-dire répondant à l'équation :

$$\varphi = e^{\theta}$$

dans laquelle $e$ = 2,71828;
- l'élément, respectivement les éléments conducteurs, sont en relief par rapport à la surface de la pièce;
- l'élément, respectivement les éléments, conducteurs sont en creux par rapport à la surface de la pièce;

- la pièce proprement dite a la forme d'une coupelle ayant une concavité devant être dirigée vers la paroi cardiaque;
- la coupelle a une section de nature à provoquer une concentration d'un champ électrique émis par les éléments conducteurs;
- la coupelle a, dans un plan médian passant par son pôle, une section courbe répondant substantiellement à l'équation :

$$r = \frac{p}{1 + \cos \varphi}$$

- les éléments conducteurs sont constitués par un dépôt d'un matériau conducteur sur une épaisseur d'au moins une couche moléculaire, dépôt obtenu notamment par micro-pulvérisation.

Grâce à l'invention, on obtient un champ électrique convergent mais non focalisé. Les lignes de force de ce champ électrique se concentrent en un tube dont on peut démontrer mathématiquement qu'il occupe dans l'espace non un point ni un segment de droite mais un volume sensiblement torique, du fait qu'il présente une singularité centrale.

L'invention sera mieux comprise par la description détaillée ci-après faite en référence au dessin annexé. Bien entendu, la description et le dessin ne sont donnés qu'à titre d'exemple indicatif et non limitatif.

La figure 1 est une vue schématique d'ensemble d'une électrode concave conforme à l'invention

La figure 2 est une vue schématique en plan d'une électrode concave conforme à l'invention.

La figure 3 est un schéma explicatif du choix optimum des spirales selon lesquelles on réalise les éléments conducteurs.

La figure 4 est une vue schématique en coupe d'une mode de réalisation de l'invention selon lequel la concavité de la coupelle est créée dans une face d'un volume sensiblement cylindrique.

La figure 5 est une vue partielle d'une électrode conforme à l'invention, montrant schématiquement un élément conducteur en creux.

La figure 6 est une vue partielle d'une électrode conforme à l'invention, montrant schématiquement un élément conducteur en relief.

La figure 7 est une vue en coupe d'une électrode concave conforme à l'invention, schématisant son effet de concentration du champ électrique.

La figure 8 est une vue schématique d'ensemble d'une électrode convexe conforme à l'invention.

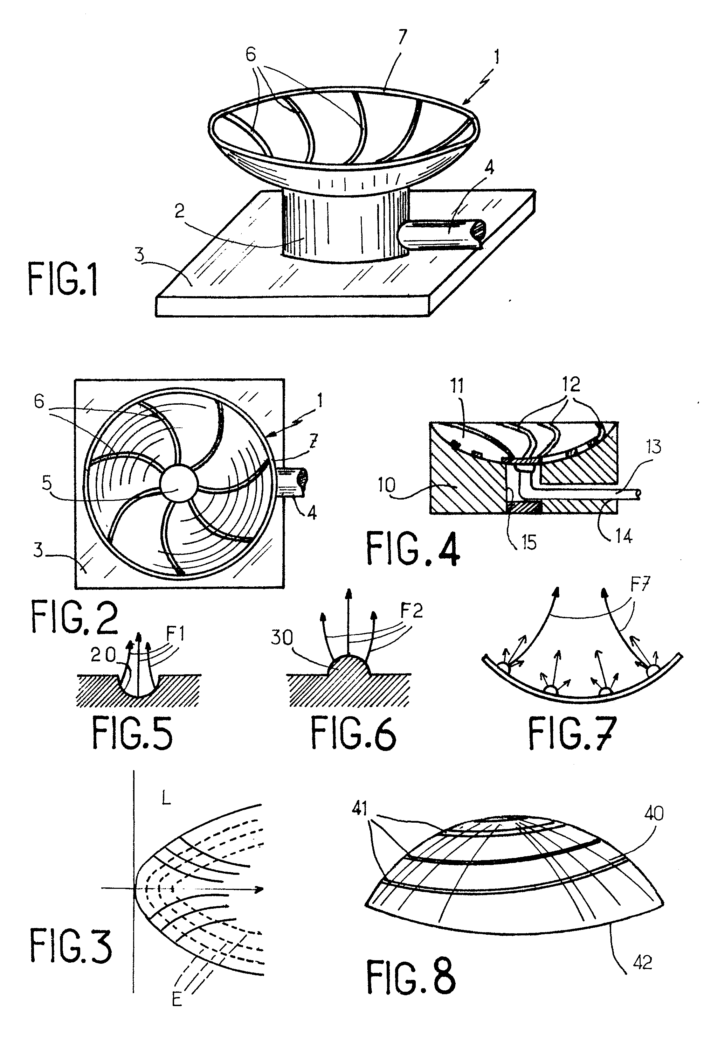

En se reportant aux figures 1 à 4, on voit une électrode conforme à l'invention qui comprend une pièce 1 en forme de coupelle concave solidaire d'un embout 2 d'une embase 3, un fil électrique 4 étant raccordé au pôle inférieur 5 de la coupelle 1. Comme cela est connu en soi, cet ensemble comporte des moyens de fixation (non représentés) permettant d'immobiliser la coupelle 1 contre la paroi du muscle cardiaque et, ici, la coupelle 1 est destinée à présenter sa concavité face à la paroi cardiaque.

Sur la face concave de la pièce 1, dite face interne, se trouvent des éléments conducteurs 6 qui sont situés hors du plan de cette face concave. En l'occurrence, ces éléments 6 sont en reliefs mais ils pourraient être en creux, comme on le précisera plus loin.

Les éléments 6 créent, quand ils sont alimentés, un champ électrique qui s'étend depuis leur surface vers l'extérieur. La forme de la concavité de la pièce 1 est choisie pour créer une concentration du champ électrique vers une zone de convergence répartie selon un volume constituant un tube de lignes de forces de champ électrique situé un peu au-delà du plan dans lequel se trouve le bord 7 de la pièce 1, c'est-à-dire sensiblement à l'endroit où doit se trouver le myocarde quand l'électrode est en place. Grâce à cette concentration, on peut obtenir des impulsions d'intensité voulue en dépensant relativement peu d'énergie. Simultanément, la forme de la pièce 1, en coupelle largement évasée offre une surface relativement vaste pour procurer un bon recueil du signal cardiaque.

Pour obtenir simultanément un seuil de stimulation peu élevé et une bonne concentration du champ électrique, on réalise la coupelle 1 en lui donnant une surface de révolution engendrée par la rotation autour d'un axe principal, d'une courbe de la famille des coniques, ayant comme équation :

$$\frac{p}{1 + \cos\varphi}$$

L'invention se propose en outre de limiter la consommation d'énergie et de minimiser l'entropie du système.

A cette fin, il faut déterminer les spirales de manière aussi performante que possible et l'on agit en tenant compte des considérations ci-après.

On sait que la spirale exponentielle $y = e^{\theta}$, avec $e = 2,71828$, base des logarithmes népériens, présente une entropie nulle. Or, elle est dans le plan, la projection d'une hélice exponentielle engendrant la surface de révolution que l'on veut donner à la coupelle 1, c'est-à-dire celle répondant à l'équation citée plus haut.

Comme les systèmes de révolution sont invariants par rotation autour de l'axe de symétrie, on peut simplifier le raisonnement en considérant une spirale dans un plan plutôt qu'une hélice dans l'espace. On négligera les effets de bord et autres singularités dont la correction peut être considérée comme négligeable ici.

Les trajectoires orthogonales de la famille de courbes
$$y^2 = 2\,(x + c)$$
dont l'équation différentielle est $y.y' = 1$ satisfont à l'équation différentielle $y' = -y$ qui admet pour solution générale :
$$y = C.e^{-x}$$
Par conséquent, la famille des courbes exponentielles est la famille des trajectoires orthogonales de la famille des courbes :
$$y^2 = 2\,(x + c)$$
et réciproquement.

En effet, en considérant l'équation :
$$y^2 = 2x + 2c$$
sa dérivée s'exprime par :
$$2\,y.y' = 2$$
soit $y.y' = 1 \Rightarrow y' = \dfrac{1}{y}$

On sait que les trajectoires orthogonales des courbes intégrales d'une équation différentielle du premier ordre sont les courbes intégrales de l'équation différentielle qui s'en déduit par le changement de $y'$ en:

$$-\frac{1}{y'}$$

La trajectoire orthogonale aux courbes du type
$$y' = \frac{1}{y}$$
relève donc de l'égalité $y' = -y$

D'où la solution :
$$y = C.\,e^{-x}$$
C'est l'équation des lignes de champ L orthogonales aux courbes d'équation :
$$y^2 = 2\,(x + c)$$
qui en constituent les isoclines, donc les lignes équipotentielles E (figure 3).

En désignant par $\underline{t}$ la variable, on peut se donner les trois fonctions suivantes :
$$x = e^t.\cos t$$
$$y = e^t.\sin t$$
$$z = e^{2t}$$

L'espace étant rapporté à un trièdre orthonormé, on vérifie que :
$$z = x^2 + y^2$$
En effet, $x^2 = e^{2t}\cos^2 t$
$$y^2 = e^{2t}\sin^2 t$$
$$z = e^{2t}\,(\cos^2 t + \sin^2 t) = e^{2t} = x^2 + y^2$$

La courbe engendrée paramétriquement par ces trois fonctions peut se projeter orthogonalement sur un plan de référence et si l'on considère dans ce plan la spirale exponentielle d'équation polaire :
$$\varphi = e^{\theta}$$
dans laquelle $\underline{e} = 2,71828$, il apparaît que les coordonnées d'un point de cette spirale sont :
$$x = e^{\theta}\cos\theta \text{ et } y = e^{\theta}\sin\theta$$

Ce point est donc la projection orthogonale dans le plan du point de la courbe correspondant à la valeur $t = $ du paramètre. Cela montre que la projection de la courbe dans le plan de référence est la spirale exponentielle d'équation polaire :
$$\varphi = e^{\theta}$$

On sait que la spirale exponentielle est un modèle de la croissance biologique : coquilles d'escargot, de nautile, etc. Une telle spirale est donc associée à l'organisation naturelle et c'est une heureuse propriété que son entropie soit nulle. Il en est de même pour le fait qu'elle soit la projection sur un plan d'une hélice exponentielle engendrant une surface de révolution à lignes de champ elles-mêmes exponentielles et directionnelles.

Ces propriétés permettent d'abaisser le seuil chronique de stimulation aux environs de 0,5 Volt et des essais ont montré que l'on peut s'approcher de 100 millivolts qui avoisine la valeur du potentiel de repos des systèmes cellulaires.

Sur la figure 4, on a représenté un mode de réalisation selon lequel l'électrode comprend une pièce 10 massive, de forme sensiblement cylindrique, dont une face d'extrémité est creusée (par usinage, forma ge, moulage etc.) pour former une concavité 11 de forme répondant aux indications données plus haut en regard des figures 1 et 2. Les éléments conducteurs 12 sont prévus en relief et peuvent être constitués, par exemple, au moyen de fils métalliques convenablement formés. Un fil électrique 13 est raccordé au centre conducteur de la concavité 11. Ainsi que cela est connu en soi, le fil 13 peut être noyé dans la masse de la pièce 1 ou être introduit grâce à des perçages 14-15.

Comme on l'a dit plus haut, les éléments conducteurs peuvent être en relief ou en creux. Sur la figure 5, on voit un élément conducteur 20 qui se présente en creux et l'on a symbolisé par des flèches F1 les composantes du champ électrique. Cela met en évidence que ces composantes sont convergentes à chaque section virtuelle du conducteur 20. Sur la figure 6, au contraire, un élément conducteur 30 est en relief et les flèches F2 qui symbolisent les composantes du champ électrique sont non plus convergentes mais divergentes. On peut penser au premier abord que la solution de la figure 5 est supérieure à celle de la figure 6. En réalité, cela n'a qu'une importance secondaire car l'essentiel est l'effet de concentration résultant, selon les flèches F3 de la figure 7.

Sur la figure 8, on a représenté un autre mode de réalisation selon lequel la pièce 40 est convexe et ne possède qu'une seule spirale 41 qui prend naissance au bord 42 et se termine au pôle 43.

L'alternative entre la concavité et la convexité de la pièce en contact avec la paroi du coeur est possible grâce au fait que la spirale en hélice se projette dans un plan, seule la concentration dans la zone du myocarde étant importante.

Il faut souligner que la pièce 1 elle-même peut être réalisée en un matériau conducteur ou pas, dès lors que les éléments conducteurs sont idividualisés car la concentration du champ électrique provient de leur conception même. Dans une pièce en matériau conducteur, les éléments sont individualisés par le fait qu'ils sont en creux ou en relief.

Sur une pièce en matériau non conducteur, les éléments peuvent être créés par dépôt d'un matériau conducteur, notamment par micropulvérisation, selon une épaisseur minime, pouvant même être réduite à une couche moléculaire.

Les spirales conçues selon les indications données plus haut et respectant raisonnablement les principes théoriques selon les impératifs de la réalité concrète, peuvent être en plus ou moins grand nombre selon les dimensions de la pièce et selon la largeur de chacune d'elles.

## Revendications

1- Electrode myocardique, du type comprenant une pièce (1-10-40) qui est conductrice au moins en partie et qui doit être placée en regard d'une paroi cardiaque, caractérisée en ce qu'elle présente au moins un élément conducteur (6-12-41) distinct de la pièce (1-10-40) proprement dite, qui s'étend hors de la surface de la pièce (1-10-40) devant être en regard de la paroi cardiaque et qui est disposé selon au moins une spirale.

2- Electrode selon la revendication 1, caractérisée en ce que la, respectivement chaque, spirale (6-12) est de type exponentiel, c'est-à-dire répondant à l'équation :

$$\varphi = e^{\Theta}$$

dans laquelle e = 2,71828

3- Electrode selon la revendication 1, caractérisée en ce que l'élément (41), respectivement les éléments conducteurs (6-12-30), sont en relief par rapport à la surface de la pièce (1-10-40).

4- Electrode selon la revendication 1, caractérisée en ce que l'élément, respectivement les éléments, conducteurs (20) sont en creux par rapport à la surface de la pièce.

5- Electrode selon la revendication 1, caractérisée en ce que la pièce proprement dite a la forme d'une coupelle (1-11) ayant une concavité devant être dirigée vers la paroi cardiaque.

6- Electrode selon la revendication 5, caractérisée en ce que la coupelle (1-11) a une section de nature à provoquer une concentration d'un champ électrique émis par les éléments conducteurs (6-12).

7- Electrode selon la revendication 5, caractérisée en ce que la coupelle (1-11) a, dans un plan médian passant par son pôle, une section courbe répondant substantiellement à l'équation :

$$r = \frac{p}{1 + \cos \varphi}$$

8- Electrode selon la revendication 1, caractérisée en ce que les éléments conducteurs (6-12-41) sont constitués par un dépôt d'un matériau conducteur sur une épaisseur d'au moins une couche moléculaire, dépôt obtenu notamment par micro-pulvérisation.

0236229

FIG.1

FIG.2

FIG.3

FIG.4

FIG.5

F1

FIG.6

F2

FIG.7

F7

FIG.8

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,Y | FR-A-2 565 111  (CELSA)<br>* Page 2, lignes 14-28; page 3, lignes 21-31; page 4, lignes 9-15; page 4, ligne 33 - page 5, ligne 4; figures 1,2,5 * | 1 | A 61 N    1/05 |
| D,A | --- | 4-8 | |
| Y | US-A-4 567 900  (J.P. MOORE)<br>* Abrégé; colonne 3, lignes 10-15; figure 5 *<br>--- | 1 | |
| A | FR-A-2 225 179  (H. LAGERGREN)<br>* Page 1, lignes 16-28; page 2, ligne 29 - page 3, ligne 12; page 3, ligne 25 - page 4, ligne 2; figure 2 *<br>--- | 1,3,8 | |
| A | EP-A-0 126 981  (MEDTRONIC INC.)<br>* Abrégé; page 5, ligne 27 - page 6, ligne 4; figure 2 *<br>--- | 1,3 | DOMAINES TECHNIQUES RECHERCHES (Int. Cl.4)<br><br>A 61 N |
| A | US-A-4 369 791  (H.G. FRIEDMAN)<br>* Abrégé; colonne 2, lignes 40-55; colonne 3, lignes 16-32; figures 1,5 *<br>--- | 1,3 | |
| P,A | FR-A-2 582 945  (NIVAROX-FAR)<br>* Page 3, lignes 2-5; page 3, ligne 33 - page 4, ligne 28; figures 1-7 *<br>--- | 1,3 | |

| Le présent rapport de recherche a été établi pour toutes les revendications | | |
|---|---|---|
| Lieu de la recherche<br>LA HAYE | Date d'achèvement de la recherche<br>11-06-1987 | Examinateur<br>RIEB K.D. |